(19) Europäisches Patentamt
European Patent Office
Office européen des brevets

(11) EP 1 867 298 A1

(12) **EUROPEAN PATENT APPLICATION**
published in accordance with Art. 158(3) EPC

(43) Date of publication:
**19.12.2007 Bulletin 2007/51**

(21) Application number: **06731273.6**

(22) Date of filing: **06.04.2006**

(51) Int Cl.:
***A61F 2/00*** *(2006.01)*

(86) International application number:
**PCT/JP2006/307325**

(87) International publication number:
**WO 2006/109668 (19.10.2006 Gazette 2006/42)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.04.2005 JP 2005110848**

(71) Applicant: **TERUMO KABUSHIKI KAISHA**
**Tokyo 151-0072 (JP)**

(72) Inventors:
- **MITSUNAGA, Seiji**
**Ashigarakami-gun,**
**Kanagawa 2590151 (JP)**
- **ISHII, Naoki**
**Ashigarakami-gun,**
**Kanagawa 2590151 (JP)**

(74) Representative: **Casalonga, Axel**
**Bureau Casalonga & Josse**
**Bayerstrasse 71/73**
**80335 München (DE)**

# (54) DRUG-ELUTING STENT SYSTEM AND METHOD OF PRODUCING DRUG-ELUTING STENT SYSTEM

(57) This invention provides a drug eluting stent system provided with a stent, which carries thereon a biologically/physiologically active substance, and a deoxidant within a package, and a manufacturing process of the drug eluting stent system. The drug eluting stent system has a substantially extended expiration date and permits a practical application.

FIG. 1

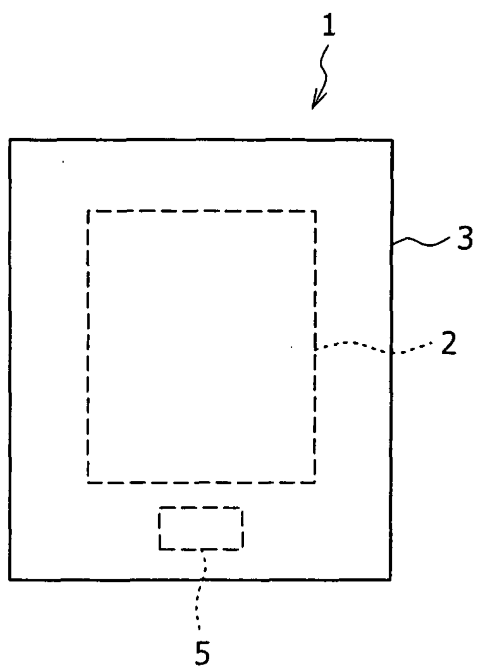




Printed by Jouve, 75001 PARIS (FR)

**Description**

Technical Field

This invention relates to a drug eluting stent system provided with a stent, which carries a biologically/physiologically active substance thereon, within a package, and also to a manufacturing process of the drug eluting stent system.

Background Art

A drug eluting stent system is provided with a stent, which carries a biologically/ physiologically active substance thereon, within a package such as a bag.
About this stent, a description will firstly be made taking as an example a case of angioplasty as applied to an ischemic heart disease.
Keeping in step with the westernization of dietary habits in Japan, there is a sharp increase in the number of patients suffering from ischemic heart diseases (angina, myocardial infarction). As a result, percutaneous transluminal coronary angioplasty (PTCA) has been performed as a method for alleviating coronary artery lesions caused by such diseases, and is rapidly finding wide-spread utility.
PTCA is a method that as will be described next, dilates the intravascular lumen at a lesion to improve the flow of blood.
Firstly, a small incision is made in the artery in the thigh or arm of a patient, and an introducer sheath (introducer device) is indwelled in place there. While causing a guide wire to take a lead through the lumen of the introducer sheath, a long hollow tube called "guide catheter" is inserted into the blood vessel.
When the lesion is located at a coronary artery, for example, the guide catheter is arranged at the entrance to the coronary artery, the guide wire is drawn out, and then, another guide wire and balloon are inserted into the lumen of the guide catheter. While causing the guide wire to take a lead, the balloon catheter is allowed to advance to the lesion in the coronary artery of the patient under radiography and the balloon is positioned at the site of the lesion.
At the position, the physician then inflates the balloon once or plural times at a predetermined pressure for 30 to 60 seconds. This procedure makes it possible to dilate the intravascular lumen of the lesion.
Owing to technological developments, such PTCA is applied to an increasingly greater variety of cases these days. In an initial stage of PTCA applications, PTCA was applied only to circumscribed lesions (of short lesion lengths) or single vessel lesions (with stenosis only at one vessel). The application of PTCA has, however, expanded to those which are located at distal sites and are eccentric and calcified, and further to multivessel lesions (with stenosis at two or more sites).
Even after the intravascular lumen of the lesion is dilated by PTCA, growth of the endangium takes place so that restenosis occurs at a rate of from 30 to 40%.
As a countermeasure for such restenosis, a stent may be employed in some instances.
Stents are tubular medical devices, which for the treatment of various diseases caused by stenosis or occlusion of blood vessels or other body ducts, can dilate the stenosed or occluded sites and can then be retained at the stenosed or occluded sites to maintain the lumens.
Many of these stents are medical devices made of a metal material or polymer material. Stents of various forms have been proposed including, for example, those obtained by providing tubular members of a metal material or polymer material with small openings and those obtained by braiding wires of a metal material or a polymer material as fibers into cylindrical forms.
When these stents are retained at stenosed or occluded sites after applying PTCA as described above, the occurrence of restenosis can be prevented to some extent.
With this measure alone, however, no pronounced effects can be exhibited.
Attempts have, therefore, been made in recent years to reduce the restenosis rate by making such a stent carry thereon a biologically/physiologically active substance such as an anticancer drug (hereinafter called the "drug") with a polymer or the like and releasing the drug from the stent over a long term locally at a site in a body duct where the stent is retained.
A drug eluting stent system is provided with a stent, which carries such a drug thereon, within a package such as a bag, and is intended to achieve the improvement, protection and the like in its delivery from a manufacturing site to an application site and the inhibition or the like of a deterioration or decomposition of the drug.
However, the expiration date of a drug eluting stent system (the period after its manufacture, during which it can be safely used without giving any deleterious effect to the body) is very short, i.e., as short as 3 months or so in many instances although the expiration date differs depending on the kind of the drug. For this short expiration date, it was difficult to practically use such drug eluting stent systems in some instances.

Disclosure of the Invention

Problem to be Solved by the Invention

The present inventor, therefore, proceeded with an investigation for a cause of the above-mentioned difficult practical application. As a result, it was found necessary for a stent with a drug carried on a surface thereof to make the drug exist widely in the form of a layer (or a film) over the surface of the stent such that the stent would exhibit significant effects in the body. It was also found that for this need, the drug on a drug eluting stent system is very prone to deterioration and decomposition compared with practically usable drug preparations such as tablets and the drug eluting stent system hence has a short expiration date in many instances.

In the case of a stent with a mixture of a drug and a polymer coated on the surface of the stent to prevent easy separation of the drug from the surface of the stent, that is, a stent provided with a biologically/physiologically active substance and a polymer, interaction between the biologically/physiologically active substance and the polymer proceeds with time so that the expiration date of the stent is shorter compared with practically-usable drug preparations such as polymer-free tablets.

Drug eluting stent systems provided with a stent, which carries a biologically/physiologically active substance thereon, within a package are accompanied by a problem in that their practical applications are difficult because their expiration dates are very short in many instances although their expiration dates differ depending on the kinds of the drugs.

An object of the present invention is, therefore, to provide a drug eluting stent system, which is provided with a significantly extended expiration date and permits a practical application even when a drug the practical application of which has heretofore been difficult is employed.

Means for Solving the Problem

The present inventor has found that in a drug stent system provided with a stent, which carries a biologically/physiologically active substance thereon, within a package, the further arrangement of a deoxidant within the package makes it possible to substantially extend its expiration date and to use it practically even when a biologically/physiologically active substance (drug) the practical application of which has heretofore been difficult is employed.

Described specifically, the present invention provides the following systems and processes (1) to (11):

(1) A drug eluting stent system comprising a stent, which carries a biologically/physiologically active substance thereon, and a deoxidant within a package.

(2) The drug eluting stent system as described above in (1), wherein the biologically/physiologically active substance is an immunosuppressor.

(3) The drug eluting stent system as described above in (2), wherein the immunosuppressor is rapamycin or a derivative thereof.

(4) The drug eluting stent system as described above in (1), wherein the biologically/physiologically active substance is an anticancer drug.

(5) The drug eluting stent system as described above in any one of (1) to (4), wherein at least a part of the package comprises a low gas-permeable material.

(6) The drug eluting stent system as described above in (5), wherein the low gas-permeable material is a film-shaped material with an aluminum foil laminated therewith.

(7) The drug eluting stent system as described above in any one of (1) to (6), wherein

the package is provided with an inner package made of a high gas-permeable material within an outer package made of a low gas-permeable material,

the deoxidant is arranged between the outer package and the inner package, and

the stent is arranged within the inner package.

(8) The drug eluting stent system as described above in (7), wherein a desiccant is further arranged between the outer package and the inner package.

(9) The drug eluting stent system as described above in (7) or (8), wherein the inner package with the stent arranged therein has been obtained by placing and sealing the stent within the inner package to provide a sealed inner package, and then sterilizing the sealed inner package by electron beam.

(10) A process for manufacturing a drug eluting stent system, which comprises:

> a first sealing step of placing and sealing a stent, which carries a biologically/physiologically active substance thereon, within an inner package made of a high gas-permeable material to provide a sealed inner package;
> a sterilization step of sterilizing the sealed inner package by electron beam to provide a sterilized inner package; and
> a second sealing step of placing and sealing the sterilized inner package and a deoxidant within an outer package made of a low gas-permeable material to provide the drug eluting stent system.

(11) The drug eluting stent system as described above in (10), wherein in the second sealing step, a desiccant is also placed within the outer package in addition to the sterilized inner package and the deoxidant.

Effects of the Invention

According to the drug eluting stent system of the present invention, it is provided with a substantially-extended expiration date and permits a practical application even when it employs a drug the expiration date of which is as short as three months or so and the practical application of which has heretofore been difficult.

Brief Description of the Drawings

[FIG. 1]
FIG. 1 is a schematic view illustrating one embodiment of a package for use in the present invention.
[FIG. 2]
FIG. 2 is a side view illustrating one embodiment of a stent for use in the present invention.
[FIG. 3]
FIG. 3 is a schematic view illustrating one embodiment of a holder tube for use in the present invention.
[FIG. 4]
FIG. 4 is a schematic side view illustrating one embodiment of a balloon catheter for use in the present invention.
[FIG. 5]
FIG. 5 is a schematic view typically illustrating one embodiment of the drug eluting stent system according to the present invention.

Description of the Reference Symbols

| | |
|---|---|
| 1 | Package, drug eluting stent system |
| 2 | Inner package |
| 3 | Outer package |
| 5 | Deoxidant |
| 6 | Desiccant |
| 10 | Stent (stent body) |
| 11 | Approximately rhomboidal element |
| 12 | Annular unit |
| 13 | Connecting member |
| 20 | Wire member |
| 30 | Holder tube |
| 100 | Balloon catheter |
| 102 | Shaft body |
| 103 | Balloon |
| 109 | Guidewire inlet port |
| 110 | Branched hub |
| 111 | Injection port |
| 112 | Inner tube |
| 113 | Outer tube |
| 113a | Distal-end-side outer tube |
| 113b | Main-side outer tube |

Best Modes for Carrying out the Invention

The present invention will hereinafter be described in detail.
A description will firstly be made about the drug eluting stent system according to the present invention. The drug eluting stent system according to the present invention is:

a system provided with a stent, which carries a biologically/physiologically active substance thereon, and a deoxidant

within a package.
More specifically, there can be mentioned, as a preferred embodiment, a system that the package is provided with an inner package made of a high gas-permeable material within an outer package made of a low gas-permeable material and is provided with the antioxidant between the outer package and the inner package, and is provided with the stent within the inner package.
As another preferred embodiment, one provided further with a desiccant between the outer package and the inner package can be mentioned.

<Package>

No particular limitation is imposed on the form of the package for use in the present invention. The package can be in any form insofar as a stent, which carries a biologically/physiologically active substance thereon, and a deoxidant can be arranged within it. For example, it can be in a form such as a bag or box.
No particular limitation is imposed either on the size of the package. When it is in the form of an approximately rectangular bag, for example, one of 300 mm × 300 mm can be used. In the case of a square bag, for example, one of 250 mm × 250 mm can be used. When it is in the form of a rectangular box, for example, one of 300 mm × 300 mm × 15 mm can be used.
On the thickness of the package, no particular limitation is imposed either. In the case of a bag-shaped package, for example, one of from 0.01 to 0.1 mm in thickness can be used.
No particular limitation is imposed either on the material of the package, although a low gas-permeable material (a material having low oxygen permeability) is preferred.
Even in the case of a package made of a high gas-permeable material (a material having high oxygen permeability), however, the gas permeability of the package becomes low when its thickness is relatively large (for example, approximately 0.5 to 1.0 mm). Such a relatively large thickness, therefore, makes it possible to use even a package made of a high gas-permeable material in the present invention.
Examples of the low gas-permeable material include aluminum, polyethylene terephthalate, polyvinyl alcohol, ethylene-vinyl alcohol copolymer, polyvinylidene chloride, and polyamides like nylons.
Examples of the high gas-permeable material include polyethylene, ethylene-vinyl acetate copolymer, polypropylene, polyvinyl chloride, and silicones.
Of these, a nonwoven fabric of polyethylene is preferred from the viewpoint of easy permeation of oxygen, which is required for electron beam sterilization, into the package.
When the drug eluting stent system according to the present invention is provided with an inner package, a nonwoven fabric of polyethylene is preferred for the inner package from the viewpoint of easy permeation of oxygen, which is required for electron beam sterilization, into the inner package.
The package for use in the present invention may preferably be formed of a film made of such a low gas-permeable material and/or a high gas-permeable material or a multilayer film (laminated film) composed of such films stacked in the form of layers as needed, because such a film or multilayer film is resistant to breakage.
It is also preferred that at least a part of the package for use in the present invention is made of a low gas-permeable material, because oxygen outside the package does not practically permeate into the package and the inclusion of a deoxidant in an amount corresponding to a volume calculated beforehand makes it possible to substantially absorb the oxygen inside the package.
The low gas-permeable material may preferably be a film-shaped material with an aluminum foil laminated therewith, because it is more difficult for oxygen on the outer side of the package to permeate to the inner side of the package.
The package for use in the present invention may preferably be formed of a multilayer film containing an aluminum film therein (aluminum pouch), because the aluminum film can be prevented from breakage.
As such an aluminum pouch, can be exemplified a multilayer film in the form of a bag, for example, of 300 mm × 300 mm and 0.1 mm in thickness and composed of a stacked structure of polyethylene terephthalate, an aluminum film and polyethylene.
As the package for use in the present invention, it is possible to use desired one of the above-described packages with another package inserted therein. In this case, the resulting package has a double structure. A further package may be placed over the package as desired to form a triple or more complex structure.
Preferred is, for example, a drug eluting stent system that such a package is provided with a double structure having a package (inner package) of a high gas-permeable material within a package (outer package) of a low gas-permeable material and the below-mentioned deoxidant is arranged within the outer package and/or inner package.
It is also possible to mention, as a further preferred embodiment, a drug eluting stent system that the package is provided with a package (inner package) of a high gas-permeable material within a package (outer package) of a low gas-permeable material.

The low gas-permeable material has the same meaning as described above.
The high gas-permeable material has the same meaning as described above.
With a drug eluting stent system of such a construction, low-dose sterilization is feasible upon placing a stent, which carries a biologically/physiologically active substance thereon, within an inner package of a high gas-permeable material and sterilizing the stent by electron beam. In this case, the inner package which has been sterilized by electron beam as described above is placed within an outer package of a low gas-permeable material to provide a double package.

When the package is provided with a package (inner package) of a high gas-permeable material within a package (outer package) of a low gas-permeable material, the outer package may preferably be made of a film-shaped material including an aluminum foil laminated therewith.

When the outer package is made of a multilayer film, no particular limitation is imposed on the order in which the respective layers are stacked together. For example, an aluminum film may be arranged preferably as an intermediate layer in the outer package from the viewpoint of ensuring the prevention of breakage of the aluminum film.

No particular limitation is imposed on the form of the outer package. The outer package can be in any form insofar as the inner package and deoxidant can be arranged within the outer package. For example, a shape such as a bag or box can be mentioned.

No particular limitation is imposed either on the size of the outer package. When it is in the form of an approximately rectangular bag, for example, one of 300 mm × 300 mm can be used. In the case of a square bag, for example, one of 250 mm × 250 mm can be used. When it is in the form of a rectangular box, for example, one of 300 mm × 300 mm × 15 mm can be used.

On the thickness of the outer package, no particular limitation is imposed either. In the case of a bag-shaped outer package, for example, one of from 0.01 to 0.1 mm can be used.

Concerning the inner package, no particular limitation is imposed on its size. It can be of any size insofar as it can be placed within the outer package.
No particular limitation is imposed either on the thickness of the inner package. In the case of a bag-shaped inner package, for example, one of from 0.01 to 0.1 mm can be used.

As a package of such a double structure, a package having a form illustrated in FIG. 1 can be mentioned.
In the exemplified package 1 of a double structure, an inner package 2 is a peelable bag of 250 × 250 mm and 0.1 mm in thickness and formed of a nonwoven fabric of polyethylene, while an outer package 3 is an aluminum pouch which is in the form of a bag of 300 × 300 mm and 0.1 mm in thickness and is composed of a stacked structure of polyethylene terephthalate, low-density polyethylene, an aluminum film, low-density polyethylene and high-density polyethylene. Further, a deoxidant 5 which will be described subsequently herein is arranged between the inner package 2 (peelable bag) and the outer package 3 (aluminum pouch).

The drug eluting stent system according to the present invention is provided with a stent, which carries a biologically/physiologically active substance thereon and will be described hereinafter, within such a package.

<Stent body>

A stent, which carries a biologically/physiologically active substance thereon and is employed in the present invention, carries the biologically/physiologically active substance on a surface of a stent body.
Firstly, a description will be made about the stent body.

No particular limitation is imposed on the material of the stent body for use in the present invention insofar as it has strength sufficient to withstand an expansion operation upon retaining the stent within a body duct. The stent body can be formed using, for example, a metal material, ceramics, a polymer material or the like.

Use of a metal material among the above-exemplified materials is preferred, because the metal material is excellent in strength and can hence exhibit an advantageous effect that the stent employed in the present invention can be surely retained at a target site within the body.

Illustrative of the metal material are stainless steel, Ni-Ti alloy, tantalum, nickel, chromium, iridium, tungsten, and cobalt alloys. In stainless steel, SUS316L is suited as its corrosion resistance is highest.

Many of stent bodies formed of such metal materials can be expanded by using balloons. Self-expanding stents can be manufactured when stent bodies are formed using a metal material such as Ni-Ti alloy having a property called "pseudoelasticity" that a strain significantly changes under a constant stress or a property that a strain gradually increases and changes as stress increases. In this case, a stent body held in a compressed form beforehand is allowed to expand by its own resilient force when its compression is released at a lesion.

Formation of a stent body with a polymer material is preferred, because the stent body exhibits advantageous effects that its flexibility is excellent and, when expanded, no excessive force is applied to a blood vessel wall.

Examples of the polymer material include polyesters such as polyethylene terephthalate and polybutylene terephthalate and polyester-based elastomers containing such polyesters as constituent units, polyamides such as nylon 6, nylon 12, nylon 66 and nylon 610 and polyamide elastomers containing such polyamides as constituent units, polyurethanes, polyolefins such as polyethylene and polypropylene and polyolefin-based elastomers containing such polyolefins as constituent units, polycarbonates such as polyethylene carbonate and polypropylene carbonate, cellulose acetate, cellulose nitrate, and the like.

Among these, polyethylene, polypropylene, polyethylene terephthalate, cellulose acetate and cellulose nitrate are preferred, because they hardly induce deposition of platelets and show no irritation to body compositions.

No particular limitation is imposed on the shape of the stent body insofar as the stent body is provided with strength sufficient to stably retain itself in a body duct. Preferred examples include one formed in a cylindrical shape by braiding wires of a metal material or fibers of a polymer material and one obtained by forming small openings in a tubular member made of a metal material or ceramics.

As a preferred example of the shape of the stent body for use in the present invention, one having the shape shown in FIG. 2 can be mentioned.

In FIG. 2, a stent body 10 is a cylindrical body, which is open at opposite end portions thereof and extends in a longitudinal direction between the opposite end portions. A side wall of the cylindrical body has numerous cutout portions communicating its outer wall and its inner wall with each other. These cutout portions are deformable to provide a structure which is expandable/contractible in the radial direction of the cylindrical body. Therefore, the cylindrical body can be retained at a target site and can retain its shape.

In the embodiment illustrated in FIG. 2, the stent body 10 is formed of wire members 20, and includes as basic units approximately rhomboidal elements 11 having cutout portions therein. A plurality of approximately rhomboidal elements 11 are connected together such that their approximately rhomboidal shapes are continuously arranged in the direction of their minor axes, thereby forming an annular unit 12. The annular unit 12 is connected with its adjacent annular units via wire-shaped connecting member 13. A plurality of annular units 12 are, therefore, continuously arranged in the direction of their axes in a partly-connected state. Owing to the construction as described above, the stent body (stent) 10 takes the form of a cylindrical body, which is open at the opposite end portions thereof and extends in the longitudinal direction between the opposite end portions. The stent body (stent) 10 has the approximately rhomboidal cutout portions, and is of the structure that it is expandable/contractible in the radial direction of the cylindrical body through deformations of the cutout portions.

When the stent body 10 is constructed of the wire members 20, the widthwise length of the wire member 20 constructed to provide the stent body 10 with numerous cutout portions may be preferably from 0.01 to 0.5 mm, more preferably from 0.05 to 0.2 mm.

The size of the stent can be selectively determined as needed depending on its application site. When used in a coronary artery of the heart, for example, its outer diameter and length before expansion may be generally from 1.0 to 3.0 mm and from 5 to 50 mm, respectively.

It is to be noted that the above-described stent 10 is merely one embodiment and that examples of the stent includes those having a wide variety of structures of which is a cylindrical body formed of wire members 20, being open at its opposite end portions and extending in a longitudinal direction between the opposite end portions, provided on its side wall with numerous cutout portions communicating its outer wall and its inner wall with each other, and being expandable/contractible in its radial direction through deformations of these cutout portions.

The stent body for use in the present invention can be either of the balloon-expanding type or of the self-expanding type. Further, the size of the stent body can be selectively determined as needed depending on its application site.

<Manufacturing process of the stent body>

No particular limitation is imposed on the manufacturing process of such a stent body, and a usual method can be applied.

As an example, a description will be made about a manufacturing process of a metal-made stent body in the form of a tubular body.

Firstly, a metal material is selected as a material for the stent body, and is molten in an inert-gas or vacuum atmosphere.

The molten metal is next cooled to form an ingot. After the ingot is mechanically polished, the ingot is formed into a large-diameter pipe by hot pressing and extrusion. By successively repeating a die drawing step and a heat treatment step, the large-diameter pipe is reduced in diameter into a pipe of predetermined wall thickness and outer diameter. A pattern of openings is adhered on a surface of the pipe, and by an etching technique such as laser etching or chemical etching, the pipe is molten except for portions covered by the pattern of openings, thereby forming openings. As an alternative, openings can also e formed by cutting the pipe along a pattern by a laser cutting technique on the basis of pattern information stored in a computer.

The tubular, metal-made stent body for use in the present invention can be manufactured by such a method as described above.

In the case of a coil-shaped stent body, for example, it can be manufactured by another method, which includes forming the ingot into a large-diameter wire by hot pressing and extrusion, successively repeating a die drawing step and a heat treatment step to reduce the large-diameter wire in diameter into a wire of predetermined thickness or outer diameter, bending the wire to apply a waveform or like pattern to the same, winding the patterned wire on a mandrel, pulling out the mandrel, and cutting the shaped wire into a predetermined length.
Other metal-made stent bodies can also be similarly manufactured by selecting metal materials as desired, melting them, and then forming the molten metals into intended shapes, respectively.
The stent for use in the present invention carries, on a surface of such a stent body, a biologically/physiologically active substance to be described next.

<Biologically/physiologically active substance>

No particular limitation is imposed on the biologically/physiologically active substance for use in the present invention and any biologically/physiologically active substance can be selected as desired, insofar as it inhibits stenosis or occlusion of a vascular system which may occur when the stent is retained at a lesion. For example, the biologically/physiologically active substance may preferably be at least one substance selected from anticancer drugs, immunosuppressors, antibiotics, antirheumatics, antithrombotics, HMG-CoA reductase inhibitors, ACE inhibitors, calcium antagonists, antihyperlipidemics, integrin inhibitors, antiallergics, antioxidants, GPIIbIIIa antagonists, retinoids, flavonoids, carotenoids, lipid-level lowering medicaments, DNA synthesis inhibitors, tyrosine kinase inhibitors, antiplatelets, antiinflammatories, tissue-derived biomaterials, interferons and NO production promoters, because it surely inhibits stenosis, restenosis or occlusion of a vascular system which may occur when the stent is retained at a lesion.
Preferred examples of the anticancer drugs include vincristine, vinblastine, vindesine, irinotecan, pirarubicin, paclitaxel, docetaxel, and methotrexate.
Preferred examples of the immunosuppressors include rapamycin and its derivatives, tacrolimus, azathioprine, cyclosporine, cyclophosphamide, mycophenolate mofetil, gusperimus, and mizoribine.
Preferred examples of the antibiotics include mitomycin, adriamycin, doxorubicin, actinomycin, daunorubicin, idarubicin, pirarubicin, aclarubicin, epirubicin, peplomycin, and zinostatin stimalamer.
Preferred examples of the antirheumatics include methotrexate, sodium thiomalate, penicillamine, and lobenzarit.
Preferred examples of the antithrombotics include heparin, aspirin, antithrombotic preparations, ticlopidine, and hirudin.
Preferred examples of the HMG-CoA reductase inhibitors include serivastatin, serivastatin sodium, atolvastatin, nisvastatin, itavastatin, fluvastatin, fluvastatin sodium, simvastatin, rosuvastatin, and pravastatin.
Preferred examples of the ACE inhibitors include quinapril, perindopril erbumine, trandolapril, cilazapril, temocapril, delapril, enalapril maleate, lisinopril, and captopril.
Preferred examples of the calcium antagonists include hifedipine, nilvadipine, diltiazem, benidipine, and nisoldipine.
Illustrative of the antihyperlipidemics is preferably probucol.
Illustrative of the integrin inhibitors is preferably AJM300.
Illustrative of the antiallergics is preferably tranilast.
Preferred examples of the antioxidants include catechins, anthocyanine, proanthocyanidin, lycopene, and β-carotene. Among the catechins, epigallocatechin gallate is particularly preferred.
Illustrative of the GPIIbIIIa antagonists is preferably abciximab.
Illustrative of the retinoids is preferably all-trans retinoic acid.
Preferred examples of the flavonoids include epigallocatechin, anthocyanine, and proanthocyanidin.
Preferred examples of the carotenoids include β-carotene and lycopene.
Illustrative of the lipid-level lowering medicaments is preferably eicosapentaenoic acid.
Illustrative of the DNA synthesis inhibitors is preferably 5-FU.
Preferred examples of the tyrosine kinase inhibitors include genistein, tyrphostin, and erbstatin.
Preferred examples of the antiplatelets include ticlopidine, cilostazol, and clopidogrel.
Preferred examples of the antiinflammatories include steroids such as dexamethasone and prednisolone.
Preferred examples of the tissue-derived biomaterials include EGF (epidermal growth factor), VEGF (vascular endothelial growth factor), HGF (hepatocyte' growth factor), PDGF (platelet derived growth factor), and BFGF (basic fibrolast growth factor).
Illustrative of the interferons is preferably interferon-γ1a.
Illustrative of the NO production promoters is preferably L-arginine.
As to whether one of these biologically/physiologically active substances or a combination of two or more different ones of these biologically/physiologically active substances should be used, a selection can be made as needed

depending on the case.

In the drug eluting stent system according to the present invention, the biologically/physiologically active substance may preferably be an immunosuppressor, because the immunosuppressor is highly effective for the inhibition of stenosis or occlusion of a vascular system.

The immunosuppressor may preferably be rapamycin or its derivative, because it is extremely effective for the inhibition of stenosis or occlusion of a vascular system.

The derivative of rapamycin is a substance with the hydroxy group (-OH) at the 40-position of rapamycin having been substituted by another functional group, and a substance represented by the following formula (1) can be mentioned as an example.

[Chemical Formula 1]

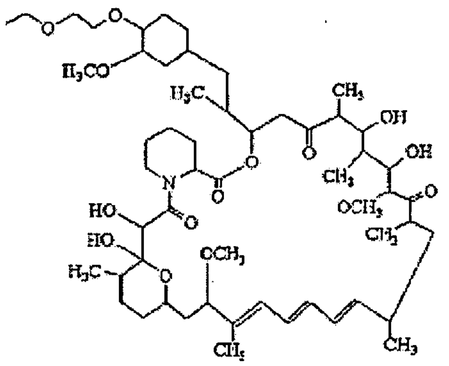

(1)

In the drug eluting stent system according to the present invention, the biologically/physiologically active substance may also be an anticancer drug preferably, because it is as effective as an immunosuppressor for the inhibition of stenosis or occlusion of a vascular system.

Such a biologically/physiologically active substance may exist in a desired form on a surface of a stent body. It is, however, preferred for the substance to exist as a mixture with a polymer to be described subsequently herein because the mixture makes it possible to gradually release the biologically/physiologically active substance into the body.

The composition ratio (mass ratio) of the biologically/physiologically active substance to the below-described polymer in such a mixture can be set at from 1:99 to 99:1, preferably at from 30:70 to 70:30, because this range makes it possible to carry the biologically/physiologically active substance as much as possible while taking the physical properties and degradability of the polymer into consideration.

Using a mixture of such a composition ratio, a layer is formed on the surface of the stent body by a method to be described subsequently herein. The thickness of the layer may be set at from 0.1 to 100 μm, preferably at from 1 to 30 μm, more preferably from 5 to 15 μm. Insofar as the layer has a thickness in this range, the stent body has merits that it can be readily inserted into a body duct such as a blood vessel and it can carry the biologically/physiologically active substance in an amount required for the treatment of a lesion.

It is to be noted that the term "the thickness of a layer" as used herein means the average thickness of the mixture on the surface of the stent.

No particular limitation is imposed on the polymer which is usable as a mixture with the biologically/physiologically active substance as described above, although one having biocompatibility or biodegradability is preferred.

No particular limitation is imposed on the polymer having biocompatibility insofar as it is equipped with biocompatibility. For example, a polyester-based resin can be used preferably, because it has biocompatibility, is equipped with certain degrees of stiffness and elasticity, and does not fall off from the surface of the stent during use (expansion) of the stent.

No particular limitation is imposed on the polyester-based resin. The polyester-based resin can be a polyester-based resin obtained by polycondensing dicarboxylic units or their derivative having ester-forming ability and diol units or their derivative having ester-forming ability to polycondensation by a known method.

No particular limitation is imposed on the polymer having biodegradability, insofar as it is a polymer which gradually degrades when the stent for use in the present invention is retained at a lesion and which does not deleteriously affect on the human or animal body. The polymer having biodegradability may preferably be at least one polymer selected from the group consisting of polyglycolic acids, polylactic acid, polycaprolactone, polyhydroxybutyric acid, cellulose,

polyhydroxybutyrate-valerate and polyorthoesters or a copolymer, mixture or compound thereof, because it has low reactivity with body tissues and its degradation within the body can be controlled.

The stent for use in the present invention may preferably carry a layer formed of a mixture of the biologically/ physiologically active substance and the polymer having biodegradability on the surface of the stent body, because the biologically/physiologically active substance is gradually released into the body to effect appropriate treatment as the polymer having biodegradability degrades.

<Deposition process of the biologically/physiologically active substance>

Such a biologically/physiologically active substance is deposited on the surface of the above-described stent body by a method as will be described next.

No particular limitation is imposed on the deposition method, and a desired method can be applied.

For example, there can be mentioned a method, which includes dissolving the biologically/physiologically active substance and the polymer at such a composition ratio as described above in a solvent such as acetone, ethanol, chloroform or tetrahydrofuran to give a solution concentration of from 0.001 to 20 wt.%, preferably from 0.01 to 10 wt.%, coating the resultant solution onto the surface of the stent body by a conventional procedure making use of a sprayer, a dispenser or the like, and then causing the solvent to evaporate.

The drug eluting stent system according to the present invention is provided with the stent, which carries thereon such a biologically/physiologically active substance as described above, and the below-described deoxidant within such a package as described above.

<Deoxidant>

No particular limitation is imposed on the kind of the deoxidant for use in the present invention. Any deoxidant can be used insofar as it has been conventionally sealed together with drugs within packages to improve their storability and also to prevent their deactivation or deterioration.

As such deoxidants, iron-based deoxidants and organic deoxidants such as ascorbic acid are known. In the present invention, both iron-based deoxidants and organic deoxidants are usable.

Depending on the functions of such deoxidants, they can be categorized into a type that absorbs only oxygen, a type that absorbs oxygen and carbon dioxide, a type that absorbs oxygen and generates carbon dioxide, and a type that absorbs carbon dioxide. These deoxidants are all usable in the present invention irrespective of their types.

These deoxidants may preferably be provided for practical applications in a form filled in bags of high gas permeability, although they can be practically used in a powder form, that is, in a generally-available form as they are. Further, those prepared into a solid preparation form such as tablets and those prepared into a film or sheet form are preferred.

No particular limitation is imposed on the size of such a deoxidant, insofar as it is of such a size as permitting its inclusion in the above-described package for use in the present invention no matter whether it is in a form filled in a bag or the like, in a solid preparation form or in a film or sheet form. The size of each deoxidant can be selectively determined as desired depending on the number of bag(s), kind, form and the like of the deoxidant, the kind of the biologically/physiologically active substance to be placed within the package, and so on.

<Manufacturing process of the deoxidant>

A description will be made about a manufacturing process of such a deoxidant.

No particular limitation is imposed on the manufacturing process of the deoxidant for use in the present invention.

When manufacturing a deoxidant, for example, in the form of tablets, shaping into a desired size can be performed in a manner known per se in the art by using, for example, an excipients (carrier), extender or the like such as diatomaceous earth, talc, montmorillonite, bentonite, kaolinite, magnesium sulfate, magnesium hydroxide, calcium carbonate or carbon black.

Upon preparing the deoxidant into a film or sheet form, on the other hand, it is only necessary, for example, to disperse or dissolve the above-described substance (component), which is usable as a deoxidant, together with optional components, i.e., a binder component such as ethylcellulose, polyvinyl alcohol or starch and a polyhydric alcohol such as glycerin or polyethylene glycol in water or an organic solvent, to impregnate a suitable film-shaped base material, for example, paper, nonwoven fabric or the like with the resulting liquid formulation or to coat the resulting liquid formulation onto a plastic film or the like by printing or the like, and then to dry the thus-impregnated base material or the thus-coated film or the like.

The drug eluting stent system according to the present invention is provided with the stent, which carries thereon the above-described biologically/physiologically active substance, and the deoxidant within the package.

The stent may be mounted, for example, on a balloon catheter and may then be placed together with the balloon catheter within the package.

More preferably, a holder tube with the balloon catheter inserted therein may be arranged within the package. Described specifically, the drug eluting stent system according to the present invention may preferably be provided with a balloon catheter and a deoxidant within the package. The balloon catheter has a stent carrying a biologically/physiologically active substance thereon and mounted on the balloon, and is inserted in a holder tube (it is to be noted that the branched hub 10 of the balloon catheter is not inserted in the holder tube). As a reason for the preference of this drug eluting stent system, the balloon catheter does not jut out of the package and can be held in a stable state within the package.

The holder tube can be a tubular member, which has a size sufficient to cover the whole balloon catheter and is equipped with a certain degree of elasticity. Such a balloon catheter can protect the internal stent and balloon catheter from external impacts or the like. As illustrated by way of example in FIG. 3, the holder tube can be wound into a vortex coil upon placing it within the package.

This is preferred.

When a holder tube is used, no particular limitation is imposed on the shape of the holder tube upon placing it within an inner package. A specific example of the shape of the holder tube upon placing it within the inner package will be described with reference to the accompanying drawing.

FIG. 3 is a schematic view illustrating one embodiment of the holder tube for use in the present invention.

As depicted by way of example in FIG. 3, the holder tube can be wound into a vortex coil upon placing it within the package. This winding of the holder tube is preferred because the balloon catheter can be retained in a stable state within the package without allowing the balloon catheter to jut out.

Preferably, the drug eluting stent system according to the present invention may be further provided with a desiccant from the viewpoint of successfully preventing decomposition or deterioration of the biologically/physiologically active substance under the influence of water.

No particular limitation is imposed on the desiccant, and silica gel can be mentioned as an example.

The arrangement of the desiccant between the outer package and the inner package can be mentioned as a preferred embodiment.

With reference to the accompanying drawings, a description will be made about a specific example of the drug eluting stent system according to the present invention. It is, however, to be noted that the drug eluting stent system according to the present invention is not limited to the specific example.

FIG. 1 is a schematic view illustrating one embodiment of the package for use in the present invention. Namely, the whole package illustrated in FIG. 1 schematically shows an embodiment of the drug eluting stent system according to the present invention.

FIG. 5 is a schematic view typically illustrating one embodiment of the drug eluting stent system according to the present invention.

Referring first to FIG. 1, the package 1 is provided with the outer package 3, the inner package 2 and the deoxidant 5. Within the outer package 3, the inner package 2 and the deoxidant 5 are arranged, and there is the deoxidant 5 between the inner package 2 and the outer package 3.

In the package 1 of the double structure shown in FIG. 1, the inner package 2 is a peelable bag of 250 mm × 250 mm and 0.1 mm in thickness formed of nonwoven polyethylene fabric, while the outer package 3 is an aluminum pouch in the form of a bag of 300 mm × 300 mm and 0.1 mm in thickness, said aluminum pouch being composed of a stacked structure of polyethylene terephthalate, low-density polyethylene, an aluminum film, low-density polyethylene, and high-density polyethylene. The deoxidant 5 is arranged between the inner package 5 and the outer package 3 (aluminum pouch).

Although the stent is not shown in FIG. 1, the stent is arranged within the inner package 2 in FIG. 1.

As a preferred embodiment, the stent may be mounted on a balloon of a balloon catheter, and except for a branched hub, the balloon catheter may be inserted in a holder tube.

Reference is next had to FIG. 5. A drug eluting stent system 1 is provided with an outer package 3, an inner package 2, a deoxidant 5 and a desiccant 6, the inner package 2, deoxidant 5 and desiccant 6 are arranged within the outer package 3, and the deoxidant 5 and desiccant 6 are arranged between the inner package 2 and the outer package 3. The inner package 2, outer package 3 and deoxidant 5 are similar to the corresponding elements in FIG. 1.

In the drug eluting stent system according to the present invention, the inner package with the stent arranged therein may preferably be one obtained by placing and sealing the stent within the inner package to provide a sealed inner package and then sterilizing the sealed inner package by electron beam. In this case, the inner package can be sterilized at low dose, thereby making it possible to avoid deteriorations or modifications of the materials of the balloon catheter and holder tube.

No particular limitation is imposed on the electron beam employed upon sterilization of the inner package insofar as the sterility of the drug eluting stent system is assured.

The dose of the electron beam upon sterilization may preferably be from 1 to 30 KGy from the viewpoint of preventing deteriorations or modifications of the materials of the balloon catheter and holder tube.
The sterilization time can be selectively determined as needed depending on the dose of the electron beam.
As a system for use in such electron beam sterilization, "RHODOTRON TT300" (manufactured by IBA) can be mentioned, for example.
The electron-beam-sterilized inner package can be used in an outer package made of a low gas-permeable material.

<Manufacturing process of the drug eluting stent system>

A manufacturing process of a preferred embodiment of the drug eluting stent system according to the present invention will next be described by using one example.
Firstly, a stent, which carries a layer of a biologically/physiologically active substance on a surface of a stent body by the above-described method, is mounted by a usual method on a balloon of such a balloon catheter as will be described subsequently herein.
The balloon catheter is next inserted into such a holder tube as will be described subsequently herein, and the holder tube with the balloon catheter inserted therein is wound into a vortex coil.
In a first sealing step, the stent with the biologically/physiologically active substance carried thereon is sealed within an inner package made of a high gas-permeable material to provide a sealed inner package. Described specifically, the holder tube is placed within a peelable bag having such a shape as the inner bag 2 in FIG. 1, and the inner package 2 is sealed.
No particular limitation is imposed on the method for sealing the inner package 2. For example, a heat sealer can be used.
After the first sealing step, the sealed inner package is sterilized by electron beam in a sterilization step to provide the sterilized inner package.
Described specifically, the stent, balloon catheter and holder tube placed in such a peelable bag are sterilized by electron beam.
The electron sterilization has the same meaning as described above.
Subsequent to the sterilization step, the sterilized inner package and a deoxidant are placed and sealed within an outer package made of a low gas-permeable material in a second sealing step to provide a drug eluting stent system.
Namely, the electron-beam-sterilized peelable bag and the deoxidant are placed and sealed together within an aluminum pouch having such a shape as the outer package in FIG. 1.
No particular limitation is imposed on the sealing method for the outer package 3. For example, a heat sealer can be used.
From the viewpoint of successfully preventing decomposition or deterioration of the biologically/physiologically active substance by water, it is preferred to also place and seal a desiccant in addition to the sterilized inner package and deoxidant within the outer package made of the low gas-permeable material in the second sealing step.
The desiccant has the same meaning as described above.
As the drug eluting stent system according to the present invention, one manufactured by such a process is preferred.
Described specifically, preferred is a drug eluting stent system provided with a balloon catheter and a deoxidant within a package, in which balloon catheter has a stent, which carries a biologically/physiologically active substance thereon, mounted on its balloon and is inserted in a holder tube.
No particular limitation is imposed on the timing at which the drug eluting stent system according to the present invention is sterilized. From the viewpoint of successfully preventing deteriorations or modifications of the balloon catheter and holder tube, however, it is preferred to subject the sealed inner package, which is internally provided with the stent, to electron-beam sterilization as described above because the inner package can be sterilized at low dose.
Conditions for sterilizing the inner package by electron beam are the same as described above.
The sterilized inner package can be arranged within the outer package.
A description will now be made about the above-described balloon catheter.
No particular limitation is imposed on a balloon catheter for use in the present invention insofar as it can be inserted in the above-described package for use in the present invention. Commonly-employed balloon catheters can each be used.
For example, a balloon catheter illustrated in FIG. 4 can be used. A description will be made about this balloon catheter.
The balloon catheter 100 illustrated in FIG. 4 is provided with a tubular shaft body 102, a foldable and inflatable balloon 103 arranged on a distal end portion of the shaft body, and a stent 10 which is mounted to cover the balloon 103 in the folded state and is caused to expand by an inflation of the balloon 103. This stent 10 is the same as the stent illustrated in FIG. 2. Further, the balloon catheter 100 is also provided with an X-ray imaging member fixed on an outer wall of the shaft body at a position corresponding to a central portion of the stent.
The shaft body 102 is provided with a balloon inflating lumen which is in communication at an end thereof with the interior of the balloon 103.
The shaft body 102 is provided with an inner tube 112, an outer tube 113, and a branched hub 110. The inner tube 112

is a tubular body internally provided with a guidewire lumen for permitting insertion of a guidewire.

The inner tube 112 can have a length of from approximately 100 to 2,000 mm, an outer diameter of from approximately 0.1 to 1.0 mm, and a wall thickness of from approximately 10 to 150 μm.

The inner tube 112 is inserted and extended in the outer tube 113, and its distal end portion protrudes from the outer tube 113. By the outer wall of the inner tube 112 and the inner wall of the outer tube 113, a balloon-inflating lumen is formed.

The outer tube 113 is a tubular body, which allows insertion and extension of the inner tube 112 therethrough and has a distal end at a position somewhat back away from the distal end of the inner tube 112.

The outer tube 113 can have a length of from approximately 100 to 2,000 mm, an outer diameter of from approximately 0.5 to 1.5 mm, and a wall thickness of from approximately 25 to 200 μm.

The outer tube 113 is formed of a distal-end-side outer tube 113a and a main-side outer tube 113b, which are joined together. The distal-end-side outer tube 113a is reduced in diameter in a tapered form at its portion on a side toward its distal end from its joined portion with the main-side outer tube 113b, and its portion on a side toward its distal end from the tapered portion has a small diameter.

The outer diameter at the small-diameter portion of the distal-end-side outer tube 113a can be from approximately 0.50 to 1.5 mm.

On the other hand, the outer diameters of a proximal end portion of the distal-end-side outer tube 113a and the main-side outer tube 113b can be from approximately 0.75 to 1.5 mm.

The balloon 103 is foldable, and in an uninflated state, the balloon 103 can be held in a folded state on and around the outer circumference of the inner tube 112.

The balloon 103 forms an expanded space between the inner wall of the balloon 103 and the outer wall of the inner tube 112. This expanded space is in communication at a rear end part thereof and over an entire circumference thereof with the inflating lumen. As the rear end of the balloon 103 is in communication with the inflating lumen of a relatively large volume as mentioned above, the injection of an inflating fluid from the inflating lumen into the balloon is assured.

As the size of the balloon 103, the outer diameter and length of a cylindrical portion (inflatable portion) when inflated can be from approximately 2 to 4 mm and from approximately 10 to 50 mm, respectively.

Between the inner tube 112 and the outer tube 113 (in the balloon-inflating lumen), a wire-shaped stiffness imparting member is inserted.

On a proximal end of the shaft body 102, the branched hub 110 is fixed.

The branched hub 110 is composed of an inner tubular hub and an outer tubular hub. The inner tubular hub has a guidewire inlet port 109, which is in communication with the guidewire lumen and defines a guidewire port, and is fixedly secured on the inner tube 112. The outer tubular hub, on the other hand, is in communication with the balloon-inflating lumen, has an injection port 111, and is fixedly secured on the outer tube 113.

No particular limitation is imposed either on the material of such a balloon catheter, although it is required to be hardly deteriorated or modified by sterilization. For example, a polyolefin, polyamide elastomer, polyester elastomer or the like can be used.

The total length of the balloon catheter illustrated by way of example in FIG. 4 (from the distal end of the inner tube 112 to the boundary between the shaft body 102 and the branched hub 110) can be from approximately 100 to 2,000 mm. Further, the cross-sectional diameter of the balloon catheter (including the stent 101) during uninflation of the balloon at the balloon 103, where the cross-sectional diameter is largest over the entire length of the balloon catheter, can be from approximately 0.6 to 2.0 mm.

Such a balloon catheter can be used in the present invention.

A description will next be made about the holder tube into which the balloon catheter is inserted.

No particular limitation is imposed on a holder tube for use in the present invention insofar as the above-described balloon catheter can be inserted.

For example, the holder tube illustrated in FIG. 3 can be used.

No particular limitation is imposed on the material of the holder tube, although it is required to be hardly deteriorated or modified by sterilization. For example, a thermoplastic resin such as a polyolefin, silicone rubber, latex rubber or the like can be used.

No limitations are imposed on the size, cross-sectional shape and the like of the holder tube, insofar as the above-descried balloon catheter can be inserted.

Preferably, the length and thickness of the holder tube can be, for example, approximately 1,480 mm and approximately 0.5 mm, respectively. When the holder tube has a substantially circular cross-sectional shape, its outer diameter can preferably be approximately 4 mm.

No particular limitation is imposed on the method of use of the drug eluting stent system according to the present invention. When the package has been sealed by a heat sealer, for example, the drug eluting stent system can be used by peeling off the sealed portion and opening the package.

A description will next be made about the process for the manufacture of a drug eluting stent system according to the present invention.

The process for manufacturing a drug eluting stent system according to the present invention, which includes:

a first sealing step of placing and sealing a stent, which carries a biologically/physiologically active substance thereon, within an inner package made of a high gas-permeable material to provide a sealed inner package;
a sterilization step of sterilizing the sealed inner package by electron beam to provide a sterilized inner package; and
a second sealing step of placing and sealing the sterilized inner package and a deoxidant within an outer package made of a low gas-permeable material to provide the drug eluting stent system.

The inner package made of the high gas-permeable material, the stent carrying the biologically/physiologically active substance thereon, the sterilization by electron beam, the deoxidant and the outer package made of the low gas-permeable material have the same meanings as described above.
As the material of the inner package, nonwoven fabric of polyethylene can be preferred from the viewpoint of readily permitting permeation of oxygen, which is required for sterilization by electron beam, into the inner package.
As the material of the outer package, an aluminum pouch formed of a stacked structure of polyethylene terephthalate, low-density polyethylene, an aluminum film, low-density polyethylene and high-density polyethylene can be preferred from the viewpoints of making the oxygen, which exists on the outer side of the outer package, difficult to permeate into the inner side of the outer package and also providing resistance to breakage.
The arrangement of the deoxidant between the inner package and the outer package makes it possible to surely absorb the oxygen within the outer package including that contained in the inner package.
Further, the arrangement of a desiccant between the inner package and the outer package makes it possible to prevent a decomposition or deterioration of the biologically/physiologically active substance under the influence of water.
The first sealing step, sterilization step and second sealing step have the same meanings as described above.
From the viewpoint of successfully preventing a decomposition or deterioration of the biologically/physiologically active substance under the influence of water, it is preferred to place and seal a desiccant in addition of the sterilized inner package and deoxidant within the outer package made of the low gas-permeable material in the second sealing step. The desiccant has the same meaning as described above.

Examples

Based on Examples, the present invention will hereinafter be described further. It should, however, be noted that the present invention is not limited to the following Examples.

<Example 1>

A biologically/physiologically active substance and a deoxidant were placed and sealed within a package. After stored at 40°C for four weeks, the remaining amount of the biologically/physiologically active substance was measured by high speed liquid chromatography (HPLC) to determine its percent residue.
The biologically/physiologically active substance, deoxidant and package used in this example were as follows.
Biologically/physiologically active substance: A rapamycin derivative represented by the following chemical formula (1) (product of Nippon Kayaku Co., Ltd.). An aliquot (0.03 g) of the rapamycin derivative was placed in a glass bottle, and further, the glass bottle was placed within the below-described package.
The biologically/physiologically active substance (the rapamycin derivative) before its placement within the package will be called "Sample A".
[Chemical Formula 2]

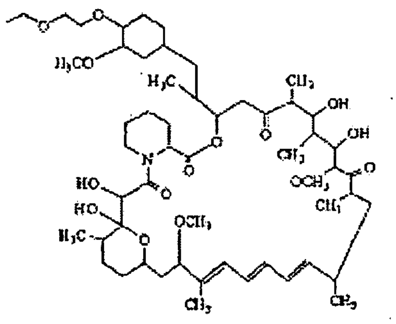

(1)

Deoxidant: "ZH-100" (product of Mitsubishi Gas Chemical Co, Inc.). Two bags, the volume of each bag being sufficient for 100 mL, were placed and sealed within the package.
Package: An aluminum pouch in the form of a bag of a size of 300 mm in length, 300 mm in width and 0.1 mm in thickness, which was formed of a stacked structure of polyethylene terephthalate, low-density polyethylene, an aluminum film, low-density polyethylene and high-density polyethylene as materials.
After stored in the above-described state at 40°C for four weeks, the biologically/physiologically active substance was taken out of the package and was provided as "Sample A1" for HPLC.
A description will next be made about a method for the HPLC measurement of the remaining amount of the rapamycin derivative in Sample A1.
Firstly, an aliquot (0.010 g; weight ($M_{A1}$)) was collected from Sample A1 and was placed in a graduated flask. Into the graduated flask, methanol was added accurately to 100 mL. The resultant solution was thoroughly stirred to uniformly dissolve Sample A1 in methanol.
An aliquot (4 mL) of the solution was collected, and placed in another graduated flask. Into the graduated flask, an internal standard substance (0.01 wt.% solution of nonyl p-hydroxybenzoate in methanol, 5 mL) was added, followed by the further addition of methanol to give a total volume of exactly 100 mL. This solution will be called "Sample Solution A1".
Using Sample A in place of Sample A1, a similar procedure was next followed to obtain "Standard Solution A". The biologically/physiologically active substance collected from Sample A at that time was 0.01 g (mass ($M_A$)).
Using Sample Solution A1 and Standard Solution A, which had been obtained by the above-described procedure, as samples, respectively, HPLC tests were conducted. Test conditions will be summarized next.

<HPLC test conditions>

Sample solution volume: 20 μL.
HPLC equipment: Manufactured by Shimadzu Corporation
Detector: Ultraviolet spectrophotometer (manufactured by Shimadzu Corporation). Measurement wavelength: 278 nm.
Column: Stainless steel tube of 4.6 mm in inner diameter and 7.5 cm in length ("J'SPHERE ODS-80H", manufactured by YMC).
Packing material: 4-μm octadecylsilylated silica gel for liquid chromatography.
Column temperature: 40°C.
Moving phase: A solution obtained by adding distilled water (550 mL) to 2-propanol (450 mL) and mixing the resultant mixture.
Flow rate: Adjusted to control the retention time of the internal standard substance at about ten minutes.
Under the above-described conditions, an HPLC test was performed on each of Sample Solution A1 and Standard Solution A to determine the ratio ($Q_T/Q_S$) of a peak area ($Q_T$) of the rapamycin derivative to a peak area ($Q_S$) of the internal standard substance.
By the following equation, the percent residual of the rapamycin derivative in Sample A1 was determined.

$$\text{Percent residual (\%)} = (M_A/M_{A1}) \times (Q_T/Q_S) \times 100$$

The results are shown in Table 1.

When Standard Solution A was subjected to an HPLC test under the above-described conditions, its separation degree was 3 or greater.
Using Standard Solution A, an HPLC test was conducted six times under the above-described conditions. The relative standard deviation of $Q_S$ determined in the respective runs was not greater than 1.5%.

<Comparative Example 1>

A test was conducted under exactly the same conditions as in Example 1 except that Sample A2, which had been obtained by storing Sample A in air at 40°C for four weeks, was used in place of Sample A1 employed above in Example 1. $M_{A2}$, the mass corresponding to $M_{A1}$ in Example 1, was 0.01 g.
The results are shown in Table 1.

<Example 2>

A stent of which a mixture of polylactic acid and the biologically/physiologically active substance had been coated on a surface was mounted and crimped on a balloon catheter. The balloon catheter with the stent mounted thereon was placed in a similar holder tube as that illustrated in FIG. 3. The holder tube was then placed within the inner package illustrated in FIG. 1, the inner package was sealed by a heat sealer, and then, electron-beam sterilization was conducted.
The inner package employed above was a bag, which was formed of nonwoven polyethylene fabric as a material and had a size of 250 mm in length, 250 mm in width and 0.1 mm in thickness.
The dose of electron beam upon sterilization of the inner package was 13.5 KGy.
The equipment used for the electron beam sterilization was "RHODOTRON TT300" (manufactured by IBA).
After the electron-beam sterilization, the inner package and deoxidant were placed in the outer package illustrated in FIG. 1, and the outer package was sealed by a heat sealer. After stored at 40°C for four weeks, the remaining amount of the biologically/physiologically active substance was measured by high speed liquid chromatography (HPLC) to determine its percent residue.
The outer package employed above was an aluminum pouch in the form of a bag of a size of 300 mm in length, 300 mm in width and 0.1 mm in thickness, which was formed of a stacked structure of polyethylene terephthalate, low-density polyethylene, an aluminum film, low-density polyethylene and high-density polyethylene as materials.
The deoxidant employed above was "ZH-100" (trade name, product of Mitsubishi Gas Chemical Company, Inc.).
The biologically/physiologically active substance and HPLC equipment (including HPLC test conditions) employed above were the same as the corresponding ones in Example 1.
The polylactic acid, stent, balloon catheter and holder tube were as follows.
Polylactic acid: Polylactic acid obtained by subjecting lactide of L-lactic acid to ring-opening polymerization, and having a glass transition point of 60°C, melting temperature (melting point) of 168°C and a weight average molecular weight of about 180,000.
Stent: Employed as the stent body was one made of SUS316L and having the same shape as that illustrated in FIG. 2. In general, after laser cutting, the diameter of a circle of its cross-section in a direction perpendicular to the direction of its length (in a radial direction) was 2.0 mm, its length was 15 mm, and its thickness was 0.08 mm. The length in the direction of the width of each wire member 20 was 0.12 mm.
A coating formulation with the polylactic acid and the rapamycin derivative dissolved at a mass ratio of 1:1 in acetone was next coated on a surface of the stent body. The solute (polylactic acid + rapamycin derivative) concentration of the coating formulation was set at 1.0 wt.%. The coating was performed by spraying the coating formulation with a sprayer ("MICROSPRAY GUN-II", manufactured by NORDSON Corporation) and then causing the acetone as a solvent to evaporate in a vacuum. As a result, approximately 450 μg of a layer of a mixture of the polylactic acid and the rapamycin derivative was confirmed to be formed on the surface of the stent body.
Balloon catheter: The balloon catheter had the same shape as that illustrated in FIG. 4. Its overall length was 1,430 mm. In the state that the stent was crimped on the balloon catheter, the outer diameter of the balloon (including the stent) was 1.1 mm.
Holder tube: The holder tube had the same shape as that illustrated in FIG. 3. Its length was 1,480 mm, and its thickness was 0.5 mm. Its cross-section is circular, and its outer diameter was 4 mm.
The stent with a mixture (called "Sample B") of such polylactic acid and biologically/physiologically active substance coated on its surface was mounted and crimped on the balloon catheter. The balloon catheter with the stent crimped thereon was placed in the holder tube. The resulting holder tube was placed and sealed within the inner package. The thus-obtained inner package was sterilized by electron beam at the above-described dose. The sterilized inner package was placed and sealed together with the deoxidant within the outer package.
The mixture of the polylactic acid and the biologically/physiologically active substance after stored in the above-described state at 40°C for four weeks was provided as "Sample B1" for HPLC.

A description will next be made about the method for the HPLC measurement of the remaining amount of the rapamycin derivative in Sample B1.

Firstly, the stent with Sample B1 carried on the surface thereof was placed together with acetone (5 mL) in a centrifuge tube, and subsequent to ultrasonication for five minutes, stirring was performed.

An aliquot (2.5 mL) of the resulting solution was then accurately collected, and was placed in a graduated flask. Into that graduated flask, the same internal standard solution (2.5 mL) as that employed in Example 1 was added, followed by the further addition of methanol to give 50 mL.

The thus-prepared solution was then filtered in its entirety through a membrane filter (thickness: 0.2 μm). The thus-obtained solution will be referred to as "Sample Solution B1".

An aliquot (0.010 g; weight ($M_{B1}$)) of Sample A employed in Example 1 was next collected, and was placed together with methanol in a graduated flask to give exactly 100 mL. Methanol was thoroughly stirred to uniformly dissolve Sample A in methanol.

An aliquot (3 mL) of the resulting solution was collected and placed in another graduated flask. Into the graduated flask, an internal standard substance (0.01 wt.% solution of nonyl p-hydroxybenzoate in methanol, 5 mL) was added, followed by the further addition of methanol to bring the whole volume to exactly 100 mL. The thus-obtained solution will be referred to as "Standard Solution B".

Using as a sample each of Sample Solution B1 and Standard Solution B obtained by the above-described procedures, an HPLC test was performed to determine the ratio ($Q_T/Q_S$) of a peak area $Q_T$ of Sample Solution B1 to a peak area $Q_S$ of Standard Solution B. Conditions for the HPLC test were the same as in Example 1.

By the following equation, the remaining amount (mass ($M_B$)) of the rapamycin derivative in Sample Solution B1 was determined.

$$M_B = M_{B1} \times (Q_T/Q_S) \times 30$$

Using the stent before its mounting on the balloon catheter as "Sample Solution B2" for HPLC, an HPLC test was performed to determine a peak area $Q_R$ of Sample Solution B2.

By the following equation, the remaining amount (mass ($M_{B2}$)) of the rapamycin derivative in Sample Solution B2 was determined.

$$M_{B2} = M_{B1} \times (Q_R/Q_S) \times 30$$

By the following equation, the percent residual of the rapamycin derivative in Sample Solution B1 was then determined.

$$\text{Percent residual (\%) of the rapamycin derivative} = (M_B/M_{B2}) \times 100$$

The results are shown in Table 1.

<Comparative Example 2>

Under exactly the same conditions as in Example 2 except that no deoxidant was placed in the outer package, a test was conducted to determine a percent residual.

The results are shown in Table 1.

Table 1

| TABLE 1 | |
|---|---|
| | Percent residual (%) |
| Example 1 | 97.3 |
| Comparative Example 1 | 18.2 |
| Example 2 | 94.2 |
| Comparative Example 2 | 62.6 |

From Table 1 and the "Guidelines for Drug Safety Tests", the biologically/physiologically active substance in the drug eluting stent system of Example 2 is expected to remain in a stable state for six months at room temperature (25°C).

As readily envisaged from the foregoing, the drug eluting stent system according to the present invention can assure a longer expiration date. Even when a drug the practical application of which has heretofore been difficult is used, the present invention permits its practical application.

## Claims

**1.** A drug eluting stent system comprising a stent, which carries a biologically/physiologically active substance thereon, and a deoxidant within a package.

**2.** The drug eluting stent system according to claim 1, wherein said biologically/physiologically active substance is an immunosuppressor.

**3.** The drug eluting stent system according to claim 2, wherein said immunosuppressor is rapamycin or a derivative thereof.

**4.** The drug eluting stent system according to claim 1, wherein said biologically/physiologically active substance is an anticancer drug.

**5.** The drug eluting stent system according to any one of claims 1 to 4, wherein at least a part of said package comprises a low gas-permeable material.

**6.** The drug eluting stent system according to claim 5, wherein said low gas-permeable material is a film-shaped material with an aluminum foil laminated therewith.

**7.** The drug eluting stent system according to any one of claims 1 to 6, wherein
said package is provided with an inner package made of a high gas-permeable material within an outer package made of a low gas-permeable material,
said deoxidant is arranged between said outer package and said inner package, and
said stent is arranged within said inner package.

**8.** The drug eluting stent system according to claim 7, wherein a desiccant is further arranged between said outer package and said inner package.

**9.** The drug eluting stent system according to claim 7 or 8, wherein said inner package with said stent arranged therein has been obtained by placing and sealing said stent within said inner package to provide a sealed inner package, and then sterilizing said sealed inner package by electron beam.

**10.** A process for manufacturing a drug eluting stent system, which comprises:

a first sealing step of placing and sealing a stent, which carries a biologically/physiologically active substance thereon, within an inner package made of a high gas-permeable material to provide a sealed inner package;
a sterilization step of sterilizing said sealed inner package by electron beam to provide a sterilized inner package;

and
a second sealing step of placing and sealing said sterilized inner package and a deoxidant within an outer package made of a low gas-permeable material to provide said drug eluting stent system.

**11.** The drug eluting stent system according to claim 10, wherein in said second sealing step, a desiccant is also placed within said outer package in addition to said sterilized inner package and said deoxidant.

FIG. 1

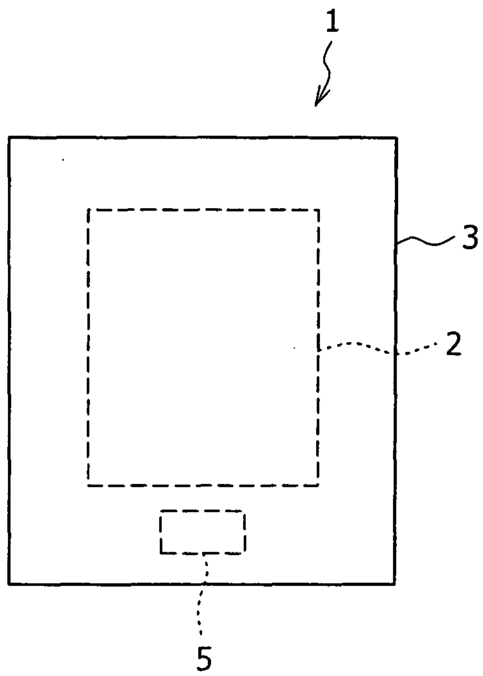

FIG. 2

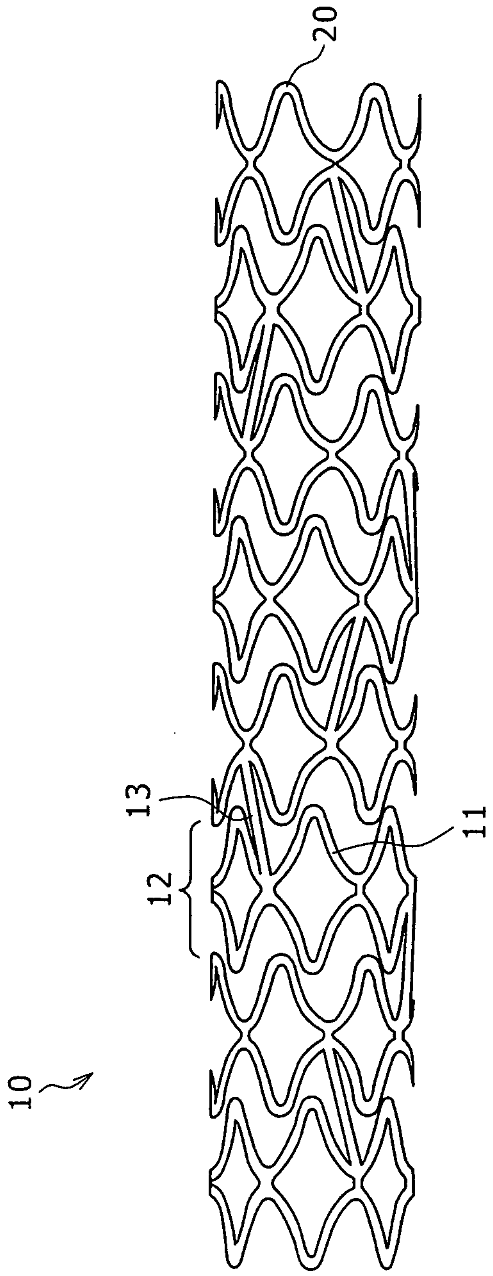

FIG. 3

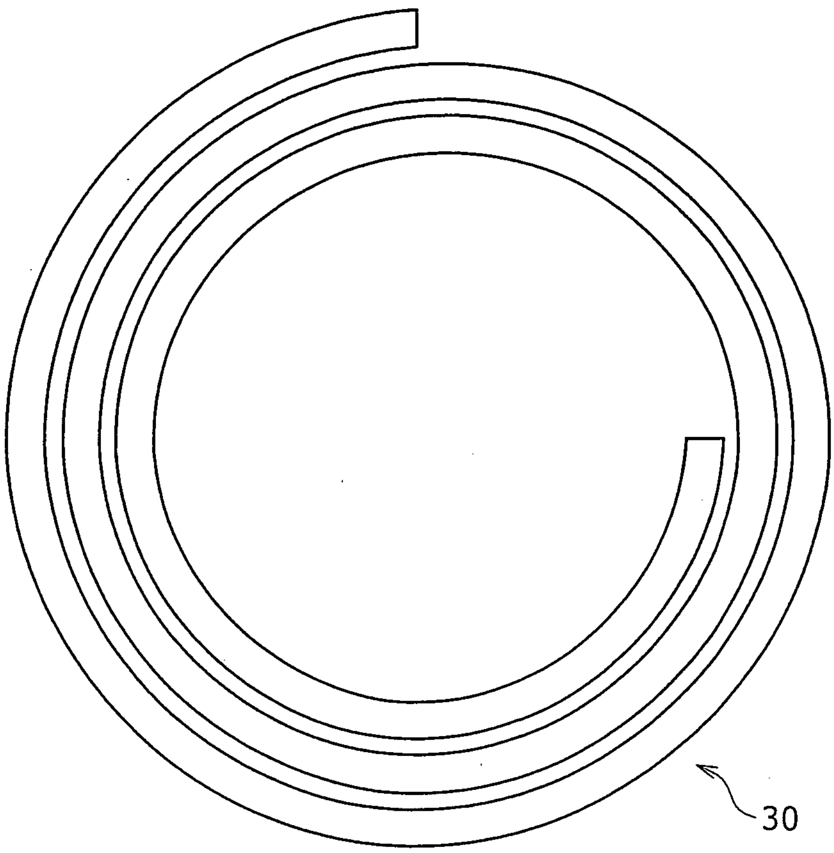

FIG.4

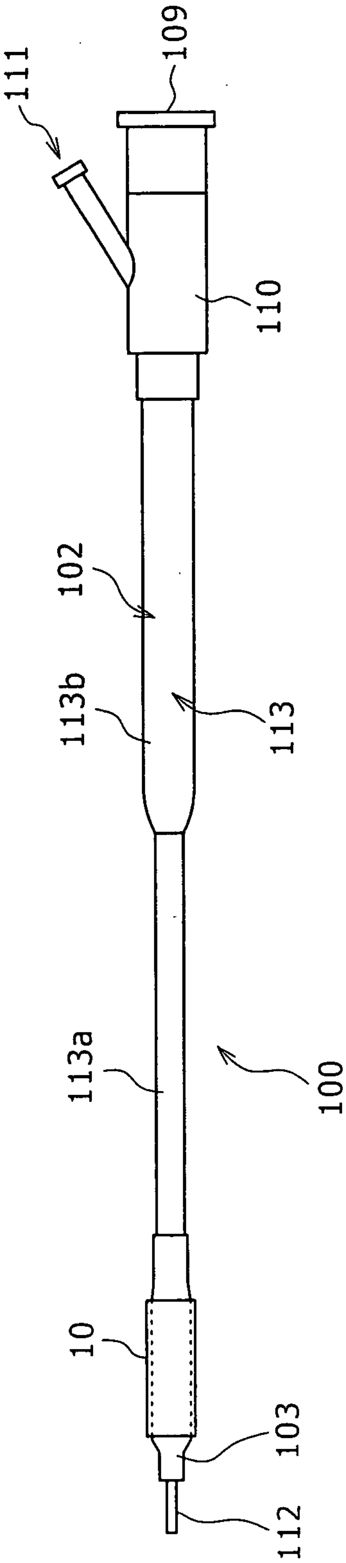

FIG. 5

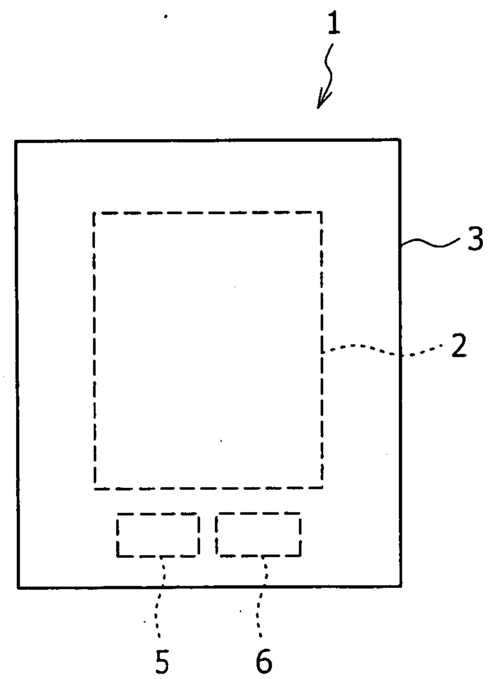

**INTERNATIONAL SEARCH REPORT**

International application No.
PCT/JP2006/307325

A. CLASSIFICATION OF SUBJECT MATTER
***A61F2/84***(2006.01), ***A61J3/00***(2006.01), ***A61L31/00***(2006.01), ***B65D65/40***(2006.01), ***B65D77/04***(2006.01), ***B65D81/24***(2006.01)

According to International Patent Classification (IPC) or to both national classification and IPC

B. FIELDS SEARCHED

Minimum documentation searched (classification system followed by classification symbols)
A61F2/84, A61J3/00, A61L31/00, B65D65/40, B65D77/04, B65D81/24

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2004/084767 A1 (AVE CONNAUGHT), 07 October, 2004 (07.10.04), Claim 1; page 3, lines 2 to 8; page 5, lines 3 to 7; Fig. 1 & JP 2006-513938 A | 1 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 July, 2006 (13.07.06) | 25 July, 2006 (25.07.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/307325

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The matter common to the inventions according to claims 1 to 11 resides in "a drug-eluting stent system which comprises a stent having a biophysiologically active substance and a deoxidizer in a package". As the results of the search, however, it is found out that the above matter is not novel because of having been disclosed in WO 2004/084767 A1. Accordingly, the above "drug-eluting stent system" falls within the category of prior art and, therefore, the above common matter cannot be considered as a special technical feature in the meaning within the second sentence of PCT Rule 13.2.

Thus, there is no matter common to all (continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/307325

Continuation of Box No.III of continuation of first sheet(2)

of the inventions according to claims 1 to 11.

Since there is no other common matter seemingly being a special technical feature in the meaning within the second sentence of PCT Rule 13.2, no technical relevancy in the meaning within PCT Rule 13 can be found out between these invention groups differing from each other. Such being the case, it is obvious that the inventions according to claims 1 to 11 do not comply with the requirement of unity of invention.

Form PCT/ISA/210 (extra sheet) (April 2005)